(19) Europäisches Patentamt
European Patent Office
Office européen des brevets

(11) **EP 3 318 245 B1**

(12) **EUROPEAN PATENT SPECIFICATION**

(45) Date of publication and mention
of the grant of the patent:
**24.02.2021 Bulletin 2021/08**

(21) Application number: **16818168.3**

(22) Date of filing: **24.06.2016**

(51) Int Cl.:
*A61K 8/29* *(2006.01)*    *A61K 8/27* *(2006.01)*
*A61K 8/02* *(2006.01)*    *A61K 8/04* *(2006.01)*
*A61Q 17/04* *(2006.01)*   *A61Q 19/00* *(2006.01)*

(86) International application number:
**PCT/KR2016/006739**

(87) International publication number:
**WO 2017/003135 (05.01.2017 Gazette 2017/01)**

(54) **UV-BLOCKING COSMETIC COMPOSITION**

UV-ABWEISENDE KOSMETISCHE ZUSAMMENSETZUNG

COMPOSITION COSMÉTIQUE BLOQUANT LES UV

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **30.06.2015 KR 20150093779**

(43) Date of publication of application:
**09.05.2018 Bulletin 2018/19**

(73) Proprietor: **Amorepacific Corporation
Seoul 140-777 (KR)**

(72) Inventors:
• **JUNG, Ha Jin
Yongin-si
Gyeonggi-do 17074 (KR)**
• **KIM, Kyung Nam
Yongin-si
Gyeonggi-do 17074 (KR)**
• **CHOI, Kyung Ho
Yongin-si
Gyeonggi-do 17074 (KR)**
• **CHOI, Yeong Jin
Yongin-si
Gyeonggi-do 17074 (KR)**

(74) Representative: **Novagraaf Technologies
Bâtiment O2
2, rue Sarah Bernhardt
CS90017
92665 Asnières-sur-Seine Cedex (FR)**

(56) References cited:
EP-A1- 1 974 717        EP-A1- 1 974 717
EP-A2- 2 724 715        WO-A2-2008/038227
KR-A- 20070 063 058     KR-A- 20080 038 622
KR-A- 20150 011 887     KR-A- 20150 011 887
KR-B1- 101 272 739      US-A1- 2011 014 254
US-A1- 2013 045 259

Remarks:
The file contains technical information submitted after
the application was filed and not included in this
specification

**Description**

[Technical Field]

The present disclosure relates to a UV blocking cosmetic composition supported in a sponge.

[Background Art]

UV blocking agents are classified into organic UV blocking agents and inorganic UV blocking agents. Since organic UV blocking agents are precipitated when they are stored for a long time and show low stability, it is difficult to use of such organic UV blocking agents in a large amount and to provide a high UV blocking effect. Therefore, the conventional UV blocking cosmetic compositions have been provided to include a large amount of inorganic UV blocking agents in order to provide a high UV blocking effect. However, in this case, the cosmetic compositions show tackiness on the skin and a soft and sticky feel of use and the consumers have expressed their displeasures during use.

In addition, as life styles have undergone a change, including generalization of leisure activities, UV blocking cosmetic compositions have been impregnated in sponges and used, and easily portable cosmetic products have gained sensational popularity. However, it has been difficult to pack such conventional UV blocking cosmetic compositions in sponges due to an increase in viscosity caused by a high content of inorganic UV blocking agents contained therein.

[References]

[Patent Document]

Korean Laid-Open Patent No. 2009-0100643

**[Disclosure]**

[Technical Problem]

A technical problem to be solved by the present disclosure is to provide a UV blocking cosmetic composition having a fresh feel of use upon the skin application, while maintaining a high UV blocking effect. Another technical problem to be solved by the present disclosure is to provide a cosmetic product, which has excellent packability when impregnated in a sponge and is easily portable.

[Technical Solution]

In one general aspect, there is provided a UV blocking cosmetic composition including titanium dioxide having a specific shape.

In another general aspect, there is provided a UV blocking cosmetic composition including titanium dioxide having a specific particle diameter.

In still another general aspect, there is provided a UV blocking cosmetic composition including titanium dioxide having a specific oil absorption.

[Advantageous Effects]

The UV blocking cosmetic composition according to the embodiments of the present disclosure includes spherical titanium dioxide microparticles having a particle diameter larger than 70 nm, and thus shows no stickiness upon skin application even when a large amount of such microparticles is contained in the composition. Therefore, the UV blocking composition has a high sun protection factor, shows a high covering effect and adhesion, and provides a fresh feel of use.

In addition, since such titanium dioxide microparticles have a low oil absorption of 0.5 or less, which is lower as compared to the other UV blocking agents, the composition can be absorbed in a sponge when it is packed in a carrier including the sponge, and thus shows high packability. Therefore, the UV blocking cosmetic composition can be provided as a formulation impregnated in a sponge and thus shows high portability.

[Description of Drawings]

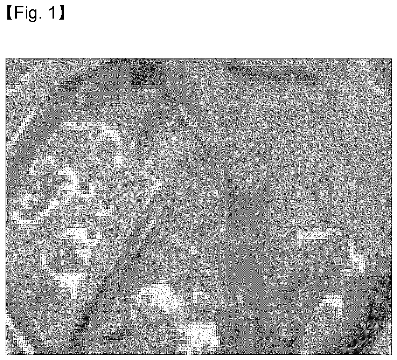

Fig. 1 is a photographic view illustrating the precipitation of the organic UV blocking agent, when Comparative Example 2 is stored under refrigeration.

[Best Mode]

As used herein, 'packability' or 'packing effect' means ability of packing a cosmetic composition in a sponge and may be expressed by the time required for packing a predetermined amount of cosmetic composition in a sponge.
As used herein, 'oil absorption' means the amount of oil required for kneading a UV blocking agent with oil so that they may be present in a hard dough state, and may be expressed by the amount (g) of oil per gram of a UV blocking agent.
Hereinafter, the present disclosure will be described in more detail.
In one aspect, there is provided a cosmetic product as claimed in claim 1 including: a UV blocking cosmetic composition including spherical titanium dioxide microparticles; and a carrier including a sponge in which the UV blocking composition is impregnated.
According to an embodiment, the spherical titanium dioxide microparticles may have a median volume particle diameter larger than 70 nm. Particularly, the titanium dioxide microparticles may have a median volume particle diameter larger than 70 nm and equal to or less than 150 nm and may have any diameter as long as the diameter allows an oil absorption maintained at 0.5 g or less. When the median volume particle diameter is larger than 150 nm, it is not possible to obtain a sufficient UV blocking effect. When the median volume particle diameter is 70 nm or less, it is not possible to obtain convenience of use and easy packability during impregnation. The term 'median volume particle diameter' means a particle diameter measured from the center of a particle. In addition, a plurality of spherical titanium dioxide microparticles may be present in the composition and each of the particles may not have the same diameter. Particularly, at least 85%, at least 90%, at least 95%, at least 99% or 100% of the spherical titanium dioxide microparticles contained in the composition may have a median volume particle diameter larger than 70 nm based on the total number of the particles.
Herein, 'oil absorption' means the amount of oil required for kneading a UV blocking agent with oil so that they may be present in a hard dough state, and may be expressed by the amount (g) of oil per gram of a UV blocking agent. According to an embodiment, the spherical titanium dioxide microparticles may have an oil absorption (g/g) larger than 0 and equal to or less than 0.5. For example, the oil absorption may be 0.5 or less, 0.49 or less, 0.48 or less, 0.47 or less or 0.46 or less, but is not limited thereto. As the surface roughness of the titanium dioxide microparticles is decreased and as the diameter of the microparticles is increased, the oil absorption may be decreased.
According to another embodiment, the titanium dioxide microparticles have an oil absorption of 0.5 or less, which is lower as compared to the other UV blocking agents. Thus, when the titanium dioxide microparticles are packed in a carrier including a sponge, they can be absorbed with ease to provide high packability.
According to still another embodiment, the UV blocking cosmetic composition may include the spherical titanium dioxide microparticles in an amount of 0.1-25 wt% based on the total weight of the composition. When the amount is larger than 25 wt%, it exceeds the maximum domestic legal limit. When the amount is less than 0.1 wt%, it is not possible to obtain a sufficient UV blocking effect.
Since the UV blocking cosmetic composition includes such spherical titanium dioxide microparticles, it is possible to improve the problems of a sticky feel of use and an unpleasant feel caused by the use of a high content of the other UV blocking agents having a different shape and/or size. Therefore, it is possible to provide a fresher feel of use while maintaining high UV blocking ability by incorporating a high content of such spherical titanium dioxide microparticles. Particularly, the UV blocking cosmetic composition may have a sun protection factor (SPF) of 30 or higher. In addition, the UV blocking cosmetic composition may have a protection grade of UVA (PA) of PA++ to PA+++.
The UV blocking cosmetic composition includes at least one of inorganic UV blocking agents and may further include organic UV blocking agents, in addition to the spherical titanium dioxide microparticles.
The inorganic UV blocking agents include at least one of non-spherical titanium dioxide particles and zinc oxide, having an oil absorption of 0.5 g-1.0 g. The term 'non-spherical' means any particle shape other than a spherical shape. For example, the non-spherical shape may include a polyhedral shape having at least two planes, rod-like shape, tubular shape and sheet-like shape.
For example, the organic UV blocking agents may include at least one selected from octylmethoxy cinnamate, ethylhexyl methoxycinnamate, octocrylene, avobenzone, butylmethoxydibenzoylmethane, oxybenzone, octyltriazone, menthyl anthranilate, 3,4-methylbenzylidene camphor and bis-ethylhexyloxyphenol methoxyphenyltriazine, but is not limited thereto.
According to still another embodiment, the UV blocking cosmetic composition may further include the inorganic UV blocking agents in an amount of 0.1-25 wt% based on the total weight of the composition.
According to still another embodiment, the UV blocking cosmetic composition may further include the organic UV blocking agents in an amount of 0.1-10 wt% based on the total weight of the composition. When the amount is less than 0.1%, it is not possible to provide a sufficient UV blocking effect. When the amount is more than 10%, it exceeds the legal limit.

Although an organic UV blocking agent, such as bis-ethylhexyloxyphenolmethoxyphenyl triazine, has a high UVA blocking effect, it is problematic in that it undergoes degradation of stability during long-term storage, resulting in precipitation, even though it is dissolved at high temperature and has increased dispersion stability. Thus, it is difficult to use the organic UV blocking agent in a large amount. Meanwhile, although an inorganic UV blocking agent, such as zinc oxide, has high long-term stability even when used in a large amount, it should be used in a large amount to provide a UVA blocking effect and thus causes tackiness on the skin and a sticky feel of use. However, since the UV blocking cosmetic composition according to an embodiment of the present disclosure uses the spherical titanium dioxide microparticles, it supplements the above-mentioned disadvantages of the organic and inorganic UV blocking agents and can provide a fresh feel of use while maintaining a high sun protection factor.

In addition, a powder-type inorganic UV blocking agent causes an increase in oil absorption and viscosity, when it is incorporated to the conventional UV blocking composition in a large amount. Thus, such an inorganic UV blocking agent cannot be packed in a sponge with ease, and it is difficult to provide the inorganic UV blocking agent as a formulation impregnated in a sponge. However, according to still another embodiment, since the spherical titanium dioxide microparticles are incorporated in combination with such a powder-type inorganic UV blocking agent, it is possible to reduce the oil absorption of the composition so that the composition may be packed in a sponge with ease, even when the powder-type inorganic UV blocking agent is incorporated in a large amount.

According to still another embodiment, the UV blocking cosmetic composition according to the present disclosure may be a liquid type composition, viscous solid type composition or a powder-containing solid type composition, particularly a liquid type composition. The formulation of the cosmetic composition may include a solution, emulsion, gel, cream, suspension or paste formulation, but is not limited thereto.

According to still another embodiment, the UV blocking cosmetic composition may include at least one of emulsion, water-in-oil (W/O) type emulsion or oil-in-water (O/W) type emulsion, oil-dispersed, water-dispersed and solubilized compositions.

According to still another embodiment, the cosmetic composition may be formulated into make-up primer, make-up base, liquid or solid foundation, concealer, lipstick, lip gloss, powder, lip liner, eye liner, mascara, eyebrow, eye shadow, blusher, twin cake, sunscreen, lotion, cream or essence, or the like, but is not limited thereto.

As used herein, the term 'carrier' means one capable of supporting any material, such as a composition, or any ingredient, and is used interchangeably with 'carrier body', 'impregnation material' or 'medium'. In addition, 'carrier' may be one used in such a manner that the material supported thereby may be discharged to a separate applicator. The composition supported in the carrier may be transferred to the skin, for example, through an application means (also referred to as an application instrument or applicator), such as a hand, puff, tip or brush.

In addition, as used herein, the term 'sponge' means a material capable of supporting any material, such as a composition, or any ingredient, includes rubber, fibers or resin formed like a sea sponge, and covers one capable of supporting and discharging a cosmetic composition. Particularly, the sponge may be a natural sponge or synthetic sponge, but is not limited thereto. For example, the natural sponge may include a sea sponge, natural rubber sponge, or the like. The synthetic sponge may include a synthetic resin sponge, urethane, foamed urethane, latex, acrylonitrile-butadiene rubber (NBR), butadiene rubber (BR), styrene-butadiene rubber (SBR), chloroprene rubber (CR), butyl rubber (isoprene-isobutylene rubber: IIR), isoprene rubber (IR), vulcanized ethylene-propylene rubber (EPR), polysulfide rubber, silicone rubber, fluororubber, urethane rubber, acrylic rubber, ethylene propylene diene monomer (EPDM) rubber, polyvinyl alcohol (PVA), ethylene vinyl acetate (EVA), nitrile rubber (NR), or the like.

As used herein, the foamed urethane as a kind of sponge may include polyether-based foamed urethane (containing ether polyol as a main base), polyester-based foamed urethane or polycarbonate-based foamed urethane, but is not limited thereto. Particularly, the foamed urethane may include polyether-based foamed urethane.

According to still another embodiment, the polyether-based foamed urethane includes polyether-based dry foamed urethane and polyether-based wet foamed urethane. The polyester-based foamed urethane includes polyester-based dry foamed urethane and polyester-based wet foamed urethane. The polycarbonate-based foamed urethane includes polycarbonate-based dry foamed urethane and polycarbonate-based wet foamed urethane.

As used herein, the term 'supportability' means the ability of receiving and retaining any material or ingredient. The supportability required for a carrier is differentiated from taking a material temporarily on an applicator in that a composition is supported homogeneously for a long time. In addition, the term 'dischargeability' or 'discharge capability' means an amount of cosmetic composition discharged when the cosmetic composition is taken out of the carrier supporting the same by using an applicator. It is preferred that an adequate amount of cosmetic composition is discharged, no more and no less than that.

As used herein, the term 'durability' means how much the foam retains its state without melting, tearing or swelling, when a cosmetic composition is supported by the foam and allowed to stand at a predetermined temperature for a predetermined time and/or how much the foam resists against repeated pressure applied by an applicator when a cosmetic composition is taken out of the foam with the applicator during use.

According to still another embodiment, the sponge may have a thickness of 1 mm-50 mm. When the sponge has a thickness less than 1 mm, the amount of cosmetic composition supported by the foam is low. When the sponge has a thickness larger than 50 mm, it is difficult to discharge the cosmetic composition during use without any residual amount of contents. The carrier using the sponge according to an embodiment may have a thickness of 3 mm-45 mm, for example, 5 mm-40 mm, such as 8 mm-35 mm, particularly 10 mm-30 mm. More particularly, the thickness may be 50 mm or less, 40 mm or less, 30 mm or less, 20 mm or less, 10 mm or less, or 5 mm or less, and 1 mm or more, 10 mm or more, 20 mm or more, 30 mm or more, 40 mm or more, or 50 mm or more.

The UV blocking cosmetic composition according to an embodiment may have a packability of 0.1-5 seconds. The term 'packability' means a time required for packing 15 g of a cosmetic composition manually in the sponge of a carrier. More particularly, the packability may be at least 0.1 sec., at least 0.5 sec., or at least 1 sec., and at most 5 sec., at most 4 sec., at most 3 sec., at most 2 sec., or at most 1 sec. As mentioned above, since the UV blocking cosmetic composition includes titanium dioxide microparticles having a decreased oil absorption, it can maintain a high sun protection factor (SPF50+, PA+++) and can be packed in a sponge at a high packing rate, even when the composition includes a high content of the microparticles.

In addition, since the cosmetic product according to an embodiment of the present disclosure includes the UV blocking cosmetic composition supported in a carrier including the sponge, the cosmetic composition can be packed well and can be supported uniformly for a long time. In addition, when the cosmetic composition is taken, an adequate amount of cosmetic composition is discharged. Further, the cosmetic product can maintain high durability for a long time. The cosmetic product according to an embodiment of the present may be provided in the form of a cosmetic container called generally 'pact', which includes a lower part capable of receiving a carrier for a cosmetic composition and an upper lid part to which a mirror may be attached. The 'pact' may further include an applicator in addition to the carrier in order to take the composition supported in the carrier and to apply the composition to the skin.

In addition, the cosmetic product according to an embodiment of the present disclosure may further include an applicator to take the cosmetic composition from the sponge carrier in which the UV blocking cosmetic composition is supported. Further, the cosmetic product may be provided in the form of a cosmetic container called generally 'pact', which includes a lower part capable of receiving a carrier for a cosmetic composition and an upper lid part to which a mirror may be attached, but is not limited thereto.

[Modes for Invention]

Exemplary embodiments now will be described more fully hereinafter. The present disclosure may, however, be embodied in many different forms and should not be construed as limited to the exemplary embodiments set forth therein.

[Example 1 and Comparative Examples 1 and 2]

UV blocking cosmetic compositions are prepared by the conventional method according to the compositions as shown in the following Table 1. Particularly, the UV blocking agents, oily ingredients and the surfactants are mixed and agitated at room temperature to a homogeneous state, and then the resultant mixture is further mixed with the thickener and agitated homogeneously. Then, the pigment is added and the resultant mixture is agitated homogeneously. In a separate mixer, the aqueous ingredients are mixed and agitated at room temperature so that they are dissolved completely. The homogeneously agitated aqueous ingredients are introduced gradually to the previously formed mixture containing the oily ingredients and the mixture is emulsified by using a homogenizer. After that, skin blocking agents and fragrances are introduced and the resultant composition is cooled to obtain each of the W/O UV blocking cosmetic compositions according to Example 1 and Comparative Examples 1 and 2.

[Table 1]

| | Ingredients (amount: wt%) | Ex. 1 | Comp. Ex. 1 | Comp. Ex. 2 |
|---|---|---|---|---|
| Oily ingredients | Dicaprylyl carbonate | 10 | 10 | 10 |
| | Decamethyl cyclopentasiloxane | 16 | 16 | 6 |
| | Phenylethyl benzoate | 0 | 0 | 10 |
| UV blocking agents | Octylmethoxy cinnamate | 7 | 7 | 3 |

(continued)

| | Ingredients (amount: wt%) | Ex. 1 | Comp. Ex. 1 | Comp. Ex. 2 |
|---|---|---|---|---|
| | Bis-ethylhexyloxyphenol methoxyphenyl triazine | 0 | 0 | 6 |
| | Titanium dioxide micorparticles A | 5 | 5 | 7 |
| | Titanium dioxide microparticles B | 10 | 0 | 0 |
| | Zinc oxide | 2 | 10 | 0 |
| Thickener | Disteardimonium hectorite | 0.2 | 0.2 | 0.2 |
| Surfactants | Sorbitan sesquioleate | 3 | 3 | 3 |
| | Lauryl PEG.PPG-18.18methicone | 2 | 2 | 2 |
| Pigments | Polymethyl methacrylate | 5 | 5 | 5 |
| | Titanium dioxide/iron oxide | 7 | 7 | 7 |
| Aqueous ingredients | Water | TO 100 | TO 100 | TO 100 |
| | Glycerin | 5 | 5 | 5 |
| | Salt | 1 | 1 | 1 |
| | Fragrance | 0.2 | 0.2 | 0.2 |

In Table 1, titanium dioxide microparticles A are non-spherical titanium dioxide microparticles having an average particle size of 15-30 nm, and titanium dioxide microparticles B are spherical titanium dioxide microparticles having an average diameter larger than 70 nm.

Hereinafter, each of the two types of titanium dioxide microparticles and another inorganic UV blocking agent, zinc oxide, is determined for the oil absorption by the test method defined in Korean Industrial Standard No. KS M ISO 787-5: 2007 (JIS K 5107-1978), standard name: "General Test Method for Pigments and Extender Pigments", Part 5: Determination of Oil Absorption"). The above-mentioned test can be identified from http://www.kssn.net/StdKS/ks_detail.asp?k1=M&k2=ISO%20787-5&k3=2, which is incorporated herein by reference.

In brief, the method for determining an oil absorption (g/g) is as follows.

1) A 1.00g of powder materials (UV blocking agent) of test object to be placed in a Petri dish.
2) The combined weight of a syringe + caprylic/capric triglyceride (CSA) is measured.
3) The syringe is used to introduce CSA of 2) to the powder material of 1) in an adequate amount.
4) The powder of 3) is mixed with oil by using a spatula.
5) Steps 2)-3) are repeated until the powder of 3) is agglomerated by the oil.
6) The amount of oil introduced herein is determined by measuring the combined weight of the syringe + CSA.

The results of determination of an oil absorption are shown in the following Table 2. The spherical titanium dioxide microparticles (titanium dioxide microparticles B) having an average diameter larger than 70 nm have an oil absorption of 0.46, which is decreased by about 20%-40% as compared to the oil absorption of the non-spherical titanium dioxide microparticles (titanium dioxide microparticles A) having an average size of 15-30 nm and that of zinc oxide.

[Table 2]

| Inorganic UV blocking agents | Oil absorption |
|---|---|
| Zinc oxide | 0.75 |
| Titanium dioxide microparticles A | 0.57 |
| Titanium dioxide microparticles B | 0.46 |

[Test Example 1] Comparison of Stability, Sun Protection Factor and Convenience of Use

Each of Example 1 and Comparative Examples 1 and 2 is evaluated for the stability, sun protection factor and usability according to the following method.

First, the stability is evaluated by storing each of the emulsion makeup cosmetic compositions of Example 1 and Comparative Examples 1 and 2 received in a plastic container in each of thermostats maintaining a specific temperature (room temperature, 45°C, 55°C, circulation of the above-mentioned temperatures, circulation of the above-mentioned temperatures and agitation, and refrigeration at 0-10°C) and observing it for at least a predetermined time (1 week, 2 weeks, 1 month, 3 months and 6 months). It is determined by the naked eyes whether each composition causes separation of the formulation or shows a band caused by separation of a pigment.

The sun protection factor (SPF) is determined according to the SPF/UVA protection test by the method for determining a sun protection effect as defined in the Cosmetic Products Regulation and the standard thereof. The UV SPF test is carried out by selecting subjects suitable for the test purpose through investigation of medical history and diagnosis of skin conditions, irradiating the back portions of the subjects to which any product is applied with UV rays with Multiport Solar UV Simulator (601-300W), and determining MED (minimal erythema dose) of the non-applied portions 16-24 hours after the UV irradiation. Three healthy females are selected as subjects and each of the cosmetic compositions of Example 1 and Comparative Examples 1 and 2 is applied to the back portions of the subjects uniformly to a thickness of 2 mg/cm$^2$. Based on MED in the non-applied portion, MED and SPF of each of standard sample and test products are determined after UV irradiation. The sun protection factor for each panel (SPFi) is calculated according to the following Mathematical Formula 1 and the sun protection factor of each sample is determined as arithmetic mean.

[Mathematical Formula 1]

Sun protection factor for each panel (SPFi) = Minimal erythema dose of product-applied portion (MEDp) / Minimal erythema dose of non-applied portion (MEDu)

The UVA protection test is carried out according to the test method described in Method for Determining UVA Protection Effect (JCIA, 2012), Japan Cosmetic Industry Association. The test method uses the method for determining UVA protection effect defined by the International Organization for Standardization (ISO24442, 2011). Each of the standard sample and the cosmetic compositions of Example 1 and Comparative Examples 1 and 2 is applied to the back portion of each subject uniformly in an amount of 2 mg/cm$^2$ and stabilized for 15 minutes. Next, UVA is irradiated based on the minimal persistent pigment darkening dose (MPPDD) of the non-applied case, and then the MPPDD and PFA of the standard sample and test product are determined. The MPPDD is judged, the judged value is used to calculate a UVA protection factor according to the following Mathematical Formula 2, and then the average is calculated. The UVA protection grades are classified as shown in the following Table 3.

[Mathematical Formula 2]

PFA = (minimal persistent pigment darkening dose (MPPDDp) of sample-applied portion) / (minimal persistent pigment darkening dose (MPPDDu) of non-applied portion)

[Table 3]

| UVA protection factor (PFA) | UVA protection grade (PA) | UVA protection effect |
| --- | --- | --- |
| Equal to or higher than 2 and less than 4 | PA+ | Effective |
| Equal to or higher than 4 and less than 8 | PA++ | Highly effective |
| Equal to or higher than 8 | PA+++ | Very highly effective |

The usability is determined by allowing twenty 25-45 aged females selected as subjects to carry out makeup with each of the cosmetic compositions of Example 1 and Comparative Examples 1 and 2 for two days. The usability is divided into non-stickiness, covering effect and adhesion and a survey is performed to give a point to each cosmetic composition based on a 3-point scale, wherein a higher point suggests a higher effect. The average points are shown in the following Table 4.

[Table 4]

| Test results | | Ex. 1 | Comp. Ex. 1 | Comp. Ex. 2 |
|---|---|---|---|---|
| Stability | Room temperature | Stable for at least 6 months | Stable for at least 6 months | Stable for at least 6 months |
| | 45°C | Stable for at least 3 months | Stable for at least 3 months | Stable for at least 3 months |
| | 55°C | Stable for at least 1 month | Stable for at least 1 month | Stable for at least 1 month |
| | Temperature circulation | Stable for at least 2 weeks | Stable for at least 2 weeks | Stable for at least 2 weeks |
| | Temperature circulation & agitation | Stable for at least 2 weeks | Stable for at least 2 weeks | Stable for at least 2 weeks |
| | Refrigeration | Stable for at least 1 month | Stable for at least 1 month | Stable for at least 1 month |
| Sun protection effect | | SPF 50.5 or more PA +++ | SPF 50.5 or more PA +++ | SPF 50.5 or more PA +++ |
| Usability | Non-stickiness | 3 | 1 | 2 |
| | Covering effect | 3 | 3 | 2 |
| | Adhesion | 2 | 3 | 1 |

After the evaluation, as shown in Table 4, Example 1 according to an embodiment of the present disclosure shows high stability and a high sun protection effect like Comparative Example 1, the conventional UV blocking agent. Example 1 shows significantly improved stability as compared to Comparative Example 2 including no titanium dioxide microparticles B and zinc oxide, when they were stored under refrigeration. Fig. 1 is a photographic view illustrating the precipitation of the organic UV blocking agent, when Comparative Example 2 is stored under refrigeration.

Further, it can be seen that a change in shape of titanium dioxide microparticles B, i.e., inorganic UV blocking agent, into a spherical shape and an increased average particle diameter larger than 70 nm according to the present disclosure result in a significant decrease in stickiness as compared to Comparative Examples 1 and 2 including the conventional titanium dioxide microparticles A and zinc oxide.

[Examples 2 and 3 and Comparative Examples 3 and 4]

UV blocking cosmetic compositions are prepared by the conventional method according to the compositions as shown in the following Table 5. Particularly, the UV blocking agents, oily ingredients and the surfactants are mixed and agitated at room temperature to a homogeneous state, and then the resultant mixture is further mixed with the thickener and agitated homogeneously. Then, the pigment and powder are added and the resultant mixture is agitated homogeneously. In a separate mixer, the aqueous ingredients are mixed and agitated at room temperature so that they are dissolved completely. The homogeneously agitated aqueous ingredients are introduced gradually to the previously formed mixture containing the oily ingredients and the mixture is emulsified by using a homogenizer. After that, the resultant composition is cooled to obtain each of the W/O UV blocking cosmetic compositions according to Examples 2 and 3 and Comparative Examples 3 and 4.

[Table 5]

| | Ingredients (amount: wt%) | Ex. 2 | Ex. 3 | Comp. Ex. 3 | Comp. Ex. 4 |
|---|---|---|---|---|---|
| Oily ingredients | Dicaprylyl carbonate | 10 | 10 | 10 | 10 |
| | Decamethyl cyclopentasiloxane | 16 | 14 | 16 | 14 |

(continued)

|  | Ingredients (amount: wt%) | Ex. 2 | Ex. 3 | Comp. Ex. 3 | Comp. Ex. 4 |
|---|---|---|---|---|---|
| UV blocking agent | Octylmethoxy cinnamate | 7 | 7 | 7 | 7 |
|  | Bis-ethyl hexyloxyphenolmethoxy phenyltriazine | 0 | 0 | 0 | 0 |
|  | Titanium dioxide microparticles A | 5 | 5 | 5 | 5 |
|  | Titanium dioxide microparticles B | 12 | 12 | 0 | 0 |
|  | Zinc oxide | 2 | 2 | 10 | 10 |
|  | Vinyldimethicone/mechiconesil sesquioxane crosspolymer | 0 | 2 | 0 | 2 |
| Thickene r | Disteardimonium hectorite | 1 | 1 | 1 | 1 |
| Surfacta nt | Sorbitan sesquioleate | 3 | 3 | 3 | 3 |
|  | Lauryl PEG.PPG-18.18methicone | 2 | 2 | 2 | 2 |
| Pigment | Polymethyl methacrylate | 5 | 5 | 5 | 5 |
|  | Titanium dioxide/iron oxide | 7 | 7 | 7 | 7 |
| Aqueous ingredien ts | Water | TO 100 | TO 100 | TO 100 | TO 100 |
|  | Glycerin | 5 | 5 | 5 | 5 |
|  | Salt | 1 | 1 | 1 | 1 |
|  | Fragrance | 0.2 | 0.2 | 0.2 | 0.2 |

Each of Examples 2 and 3 and Comparative Examples 3 and 4 is determined for the viscosity right after the preparation and after storing it for 1 day at room temperature, and the results are shown in the following Table 6. Herein, the viscosity is determined by using LVDV II+PRO with spindle No. 64 at a spindle speed of 12 rpm.

[Table 6]

| Viscosity (centi poise, cps) | Ex. 2 | Ex. 3 | Comp. Ex. 3 | Comp. Ex. 4 |
|---|---|---|---|---|
| Right after preparation | 21867 | 24500 | 24533 | 28800 |
| 1 day after preparation | 15467 | 18867 | 17600 | 25600 |

It can be seen from the above results that Example 2 according to an embodiment of the present disclosure has a lower viscosity as compared to Comparative Example 3 including conventional titanium dioxide microparticles A according to the related art as titanium dioxide and zinc oxide, by virtue of a change in shape of an inorganic UV blocking agent into a spherical shape and an increased average particle diameter larger than 70 nm. In addition, Example 3 including powdery vinyl dimethicone/methiconesilsesquioxane crosspolymer has a lower viscosity as compared to Comparative Example 4.

[Test Example 2] Comparison of Packability

The packability is determined by the time where 15g of the cosmetic composition is packed manually in urethane foam (packed by using a spatula after a carrier is introduced to a container). Particularly, determined is the time required for packing 15 g of each of the UV blocking cosmetic compositions according to Examples 2 and 3 and Comparative Examples 3 and 4 manually in urethane foam having a size of a circular diameter 44 mm x height 9.0 mm.

[Table 7]

|  | Ex. 2 | Ex. 3 | Comp. Ex. 3 | Comp. Ex. 4 |
|---|---|---|---|---|
| Packability | 1-3 sec | 2-3 sec | 1-3 sec | 5 sec or more |

As can be seen from the above results, the cosmetic compositions of Examples 2 and 3 according to the present disclosure show a packability corresponding to 1-3 seconds, which suggests that the cosmetic compositions can be packed easily in a sponge such as urethane foam. Particularly, Example 3 shows a packability improved by about 2 times or more than Comparative Example 4, even though it includes powder. It is thought that such a result is obtained by virtue of a reduced oil absorption of 0.5 or less through the incorporation of spherical titanium dioxide microparticles having an average particle diameter larger than 70 nm and a decrease in viscosity of the composition.

While the exemplary embodiments have been shown and described, it will be understood by those skilled in the art that various changes in form and details may be made thereto without departing from the scope of this disclosure as defined by the appended claims.

**Claims**

1.   A cosmetic product comprising:

an UV blocking cosmetic composition comprising:

- an inorganic UV blocking agent comprising spherical titanium dioxide microparticles having a median volume particle diameter larger than 70 nm and equal to or less than 150 nm and an oil absorption larger than 0 and equal to or less than 0.5,
- a further inorganic UV blocking agent comprising non-spherical titanium dioxide microparticles and zinc oxide, and

a carrier including a sponge in which the UV blocking composition is impregnated,
wherein the oil absorption is expressed by the amount of oil per gram of a UV-blocking agent determined by the test method defined in Korean Industrial Standard No. KS M ISO 787-5:2007, wherein the oil is caprylic/capric triglyceride, and
wherein the non-spherical titanium dioxide microparticles and zinc oxide has an oil absorption of 0.5-1.0,
wherein the shape of the non-spherical titanium dioxide microparticles comprises at least one shape selected from a polyhedral shape having at least two planes, rod-like shape, tubular shape and sheet-like shape.

2.   The cosmetic product according to claim 1, wherein the UV blocking cosmetic composition comprises the spherical titanium dioxide microparticles in an amount of 0.1-25 wt% based on the total weight of the composition.

3.   The cosmetic product according to claim 1 or 2, wherein the UV blocking cosmetic composition further comprises organic UV blocking agents.

4.   The cosmetic product according to claim 3, wherein
the organic UV blocking agent comprises at least one organic UV blocking agent selected from octylmethoxy cinnamate, ethylhexyl methoxycinnamate, octocrylene, avobenzone, butylmethoxydibenzoylmethane, oxybenzone, octyltriazone, menthyl anthranilate, 3,4-methylbenzylidene camphor and bis-ethylhexyloxyphenol methoxyphenyltriazine,

5.   The cosmetic product according to claim 1-4, wherein the UV blocking cosmetic composition further comprises the further inorganic UV blocking agent in an amount of 0.1-25 wt% based on the total weight of the composition.

6.   The cosmetic product according to any one of claims 3-5, wherein the UV blocking cosmetic composition further comprises the organic UV blocking agent in an amount of 0.1-10 wt% based on the total weight of the composition.

7.   The cosmetic product according to any one of claims 1-6, wherein the UV blocking cosmetic composition is selected from the group consisting of a solution, emulsion, gel, cream and suspension.

8. The cosmetic product according to any one of claims 1-7, wherein the sponge comprises at least one selected from the group consisting of natural rubber, synthetic resins, polyurethane, latex, acrylonitrile-butadiene rubber (NBR), butadiene rubber (BR), styrene-butadiene rubber (SBR), chloroprene rubber (CR), butyl rubber (isoprene-isobutylene rubber: IIR), isoprene rubber (IR), vulcanized ethylene-propylene rubber (EPR), polysulfide rubber, silicone rubber, fluororubber, urethane rubber, acrylic rubber, ethylene propylene diene monomer (EPDM) rubber, polyvinyl alcohol (PVA), ethylene vinyl acetate (EVA), nitrile rubber (NR), or the like.

9. The cosmetic product according to any one of claims 1-8, wherein the UV blocking cosmetic composition has a packability of 0.1-5 seconds, and the packability is defined as time required for packing 15 g of a cosmetic composition manually in the sponge of a carrier.

10. The cosmetic product according to any ones of claims 1-9, which further comprises an applicator with which the cosmetic composition is taken out of the carrier impregnated with the UV blocking cosmetic composition.

**Patentansprüche**

1. Ein kosmetisches Produkt, umfassend:

   eine UV-blockierende kosmetische Zusammensetzung, umfassend:

   - ein anorganisches UV-Blockierungsmittel, umfassend kugelförmige Titandioxid-Mikropartikel mit einem mittleren Volumenteilchendurchmesser größer als 70 nm und gleich oder kleiner als 150 nm und einer Ölabsorption größer als 0 und gleich oder kleiner als 0,5,
   - ein weiteres anorganisches UV-Blockierungsmittel, das nicht-kugelförmige Titandioxid-Mikropartikel und Zinkoxid umfasst, und

   einen Träger, der einen Schwamm einschließt, in den die UV-blockierende Zusammensetzung imprägniert ist, wobei die Ölabsorption durch die Menge an Öl pro Gramm eines UV-blockierenden Mittels ausgedrückt wird, bestimmt durch das Testverfahren, das im koreanischen Industriestandard Nr. KS M ISO 787-5:2007 definiert ist, wobei das Öl Capryl- /Caprinsäuretriglycerid ist, und wobei die nicht-kugelförmigen Titandioxid-Mikropartikel und das Zinkoxid eine Ölabsorption von 0,5-1,0 haben, wobei die Form der nicht-kugelförmigen Titandioxid-Mikropartikel mindestens eine Form umfasst, die aus einer polyedrischen Form mit mindestens zwei Ebenen, einer stabförmigen Form, einer röhrenförmigen Form und einer blattförmigen Form ausgewählt ist.

2. Kosmetisches Produkt nach Anspruch 1, wobei die UV-blockierende kosmetische Zusammensetzung die sphärischen Titandioxid-Mikropartikel in einer Menge von 0,1-25 Gew.-%, bezogen auf das Gesamtgewicht der Zusammensetzung, umfasst.

3. Das kosmetische Produkt nach Anspruch 1 oder 2, wobei die UV-blockierende kosmetische Zusammensetzung weiterhin organische UV-blockierende Mittel umfasst.

4. Das kosmetische Produkt nach Anspruch 3, wobei das organische UV-Blockierungsmittel mindestens ein organisches UV-Blockierungsmittel umfasst, ausgewählt aus Octylmethoxycinnamat, Ethylhexylmethoxycinnamat, Octocrylen, Avobenzon, Butylmethoxydibenzoylmethan, Oxybenzon, Octyltriazon, Menthylanthranilat, 3,4-Methylbenzylidenkampfer und Bis-Ethylhexyloxyphenolmethoxyphenyltriazin,

5. Kosmetisches Produkt nach Anspruch 1-4, wobei die UV-blockierende kosmetische Zusammensetzung weiterhin das weitere anorganische UV-blockierende Mittel in einer Menge von 0,1-25 Gew.-%, bezogen auf das Gesamtgewicht der Zusammensetzung, umfasst.

6. Kosmetisches Produkt nach einem der Ansprüche 3 bis 5, wobei die UV-blockierende kosmetische Zusammensetzung weiterhin das organische UV-blockierende Mittel in einer Menge von 0,1 bis 10 Gew.-%, bezogen auf das Gesamtgewicht der Zusammensetzung, umfasst.

7. Das kosmetische Produkt nach einem der Ansprüche 1-6, wobei die UV-blockierende kosmetische Zusammensetzung ausgewählt ist aus der Gruppe bestehend aus einer Lösung, Emulsion, Gel, Creme und Suspension.

8. Das kosmetische Produkt nach einem der Ansprüche 1-7, wobei der Schwamm mindestens einen umfasst, der ausgewählt ist aus der Gruppe bestehend aus Naturkautschuk, synthetischen Harzen, Polyurethan, Latex, Acrylnitril-Butadien-Kautschuk (NBR), Butadien-Kautschuk (BR), Styrol-Butadien-Kautschuk (SBR), Chloropren-Kautschuk (CR), Butylkautschuk (Isopren-Isobutylen-Kautschuk: IIR), Isopren-Kautschuk (IR), vulkanisierter Ethylen-Propylen-Kautschuk (EPR), Polysulfid-Kautschuk, Silikon-Kautschuk, Fluorkautschuk, Urethan-Kautschuk, Acryl-Kautschuk, Ethylen-Propylen-Dien-Monomer-Kautschuk (EPDM), Polyvinylalkohol (PVA), Ethylen-Vinylacetat (EVA), Nitril-Kautschuk (NR) oder dergleichen.

9. Kosmetisches Produkt nach einem der Ansprüche 1 bis 8, wobei die UV-blockierende kosmetische Zusammensetzung eine Packbarkeit von 0,1 bis 5 Sekunden aufweist und die Packbarkeit als Zeit definiert ist, die erforderlich ist, um 15 g einer kosmetischen Zusammensetzung manuell in den Schwamm eines Trägers zu packen.

10. Kosmetisches Produkt nach einem der Ansprüche 1-9, **dadurch gekennzeichnet, dass** es ferner einen Applikator umfasst, mit dem die kosmetische Zusammensetzung aus dem mit der UV-blockierenden kosmetischen Zusammensetzung imprägnierten Träger entnommen wird.

## Revendications

1. Un produit cosmétique comprenant :

    une composition cosmétique bloquant les UV comprenant :

       - un agent inorganique de blocage des UV comprenant des microparticules sphériques de dioxyde de titane ayant un diamètre volumique médian des particules supérieur à 70 nm et inférieur ou égal à 150 nm et une absorption d'huile supérieure à 0 et inférieure ou égale à 0,5,
       - un autre agent inorganique de blocage des UV comprenant des microparticules non sphériques de dioxyde de titane et de l'oxyde de zinc, et

    un support comprenant une éponge dans laquelle est imprégnée la composition bloquant les UV,
    où l'absorption d'huile est exprimée par la quantité d'huile par gramme d'un agent bloquant les UV déterminée par la méthode d'essai définie dans la norme industrielle coréenne n° KS M ISO 787-5:2007, où l'huile est un triglycéride caprylique/caprique, et
    dans laquelle les microparticules de dioxyde de titane non sphériques et l'oxyde de zinc ont une absorption d'huile de 0,5 à 1,0,
    dans laquelle la forme des microparticules de dioxyde de titane non sphériques comprend au moins une forme choisie parmi une forme polyédrique ayant au moins deux plans, une forme de tige, une forme tubulaire et une forme de feuille.

2. Produit cosmétique selon la revendication 1, dans lequel la composition cosmétique bloquant les UV comprend des microparticules sphériques de dioxyde de titane en une quantité de 0,1 à 25 % en poids par rapport au poids total de la composition.

3. Produit cosmétique selon la revendication 1 ou 2, dans lequel la composition cosmétique bloquant les UV comprend en outre des agents organiques bloquant les UV.

4. Le produit cosmétique selon la revendication 3, dans lequel
    l'agent organique de blocage des UV comprend au moins un agent organique de blocage des UV choisi parmi l'octylméthoxy cinnamate, l'éthylhexyl méthoxycinnamate, l'octocrylène, l'avobenzone, le butylméthoxydibenzoylméthane, l'oxybenzone, l'octyltriazone, l'anthranilate de menthyle, le 3,4-méthylbenzylidène camphre et la bis-éthylhexyloxyphénol méthoxyphényltriazine,

5. Produit cosmétique selon les revendications 1 à 4, dans lequel la composition cosmétique de blocage des UV comprend en outre l'agent inorganique de blocage des UV en une quantité de 0,1 à 25 % en poids par rapport au poids total de la composition.

**6.** Produit cosmétique selon l'une quelconque des revendications 3 à 5, dans lequel la composition cosmétique de blocage des UV comprend en outre l'agent organique de blocage des UV en une quantité de 0,1 à 10 % en poids sur la base du poids total de la composition.

**7.** Produit cosmétique selon l'une quelconque des revendications 1 à 6, dans lequel la composition cosmétique bloquant les UV est choisie dans le groupe constitué par une solution, une émulsion, un gel, une crème et une suspension.

**8.** Produit cosmétique selon l'une quelconque des revendications 1 à 7, dans lequel l'éponge comprend au moins un élément choisi dans le groupe constitué par le caoutchouc naturel, les résines synthétiques, le polyuréthane, le latex, le caoutchouc acrylonitrile-butadiène (NBR), le caoutchouc butadiène (BR), le caoutchouc styrène - butadiène (SBR), le caoutchouc chloroprène (CR), le caoutchouc butyle (caoutchouc isoprène-isobutylène) : IIR), caoutchouc isoprène (IR), caoutchouc éthylène-propylène vulcanisé (EPR), caoutchouc polysulfure, caoutchouc silicone, caoutchouc fluoré, caoutchouc uréthane, caoutchouc acrylique, caoutchouc éthylène-propylène-diène monomère (EPDM), alcool polyvinylique (PVA), éthylène-acétate de vinyle (EVA), caoutchouc nitrile (NR), ou similaires.

**9.** Produit cosmétique selon l'une quelconque des revendications 1-8, dans lequel la composition cosmétique bloquant les UV a une aptitude au conditionnement de 0,1-5 secondes, et l'aptitude au conditionnement est définie comme le temps nécessaire pour emballer 15 g d'une composition cosmétique manuellement dans l'éponge d'un support.

**10.** Le produit cosmétique selon l'une quelconque des revendications 1 à 9, qui comprend en outre un applicateur avec lequel la composition cosmétique est retirée du support imprégné de la composition cosmétique bloquant les UV.

【Fig. 1】

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 20090100643